**Europäisches Patentamt**

**European Patent Office**

**Office européen des brevets**

(19)

(11) Publication number : **0 511 784 A1**

# (12) EUROPEAN PATENT APPLICATION

(21) Application number : **92303687.5**

(51) Int. Cl.$^5$ : **C12P 19/06, C04B 35/66**

(22) Date of filing : **24.04.92**

(30) Priority : **26.04.91 US 691946**

(43) Date of publication of application :
**04.11.92 Bulletin 92/45**

(84) Designated Contracting States :
**AT BE CH DE DK ES FR GB GR IT LI LU NL PT SE**

(71) Applicant : **MERCK & CO. INC.**
**126, East Lincoln Avenue P.O. Box 2000**
**Rahway New Jersey 07065-0900 (US)**

(72) Inventor : **Talashek, Todd**
**9950 Scripps Westview No. 71**
**San Diego, CA 92131 (US)**
Inventor : **Cleary, Joseph M.**
**8042 Lake Adlon Drive**
**San Diego, CA 92119 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Low-ash xanthan gum.**

(57)    A low-ash xanthan gum and process for its preparation. The gum gives a reduced level of inorganic residue upon combustion, and is particularly useful as a stabilizer in ceramic formulations.

EP 0 511 784 A1

## BACKGROUND OF THE INVENTION

Xanthan gum is a water-soluble biopolymer which is produced exocellularly by a microorganism, <u>Xanthomonas</u> <u>campestris</u>. Its unusual properties make it useful in oil well drilling muds, as a viscosity control additive in secondary recovery of petroleum by water flooding, and as a stabilizer, emulsifier and thickener in foods and as a suspending agent in numerous industrial applications. (Encyclopedia of Polymer Science and Engineering 90l-9l8 <u>l7</u> IInd Ed (l989) John Wiley & Sons).

Standard fermentation of <u>Xanthomonas</u> <u>campestris</u> on a continuous or batch basis gives a viscous broth or beer which contains the desired gum. Xanthan gum recovered from standard fermentation broth by alcohol precipitation has approximately 9-l0% ash. The ash comprises the counterions (potassium, sodium, calcium and magnesium) associated with this anionic polysaccharide and occluded salts from the fermentation broth. For certain applications it is desirable to prepare xanthan gum having low ash content.

## SUMMARY OF THE INVENTION

The invention is a low-ash xanthan gum and method for preparing low-ash xanthan gum. <u>Xanthomonas</u> <u>campestris</u> is grown in aqueous media comprising carbohydrate, a nitrogen source and a minimum amount of inorganic ions necessary for cell growth and gum production. The ash content is less than 2%, preferably less than l%. During fermentation and xanthan gum production, the broth is kept at neutral pH with a suitable water soluble organic base, referably ammonium hydroxide. After fermentation, the broth is pasteurized and low-ash xanthan gum is recovered by precipitation with alcohol, drying and milling.

Alternatively or additionally, ammonium bicarbonate or other ammonium salts of organic acids is added to the broth prior to precipitation or to the alcohol precipitated presscake prior to drying.

## DETAILED DESCRIPTION OF THE INVENTION

Prior art fermentations of xanthan gum do not generate low-ash xanthan gum. Typical fermentations are described in the following United States Patents: 4,375,5l2; 4,352,882; 4,3l6,0l2; 3,67l,398; 3,594,280; 3,59l,578; 3,48l,889; 3,433,708; 3,39l,06l; 3,39l,060; 3,27l,267; and 3,020,206, and in United Kingdom Patent l,448,645

Production of low-ash xanthan gum according to the present invention involves fermentation of <u>Xanthomonas</u> <u>campestris</u>, NRRL B-1459, using certain fermentation conditions and procedures known in the art as well as special fermentation conditions and procedures for inducing formation of low-ash xanthan gum. The method of the invention is applicable to both batch and continuous fermentations. In a conventional batch fermentation, the reaction is carried out until all the substrate is utilized or the reaction mass is too viscous for further agitation. Normally, the reaction is stopped and the product recovered at this point.

Low-ash xanthan gum is produced using <u>Xanthomonas</u> <u>campestris</u> bacteria, including strain NRRL B-l459 and mutant strains capable of producing xanthan gum.

During the fermentation of xanthan gum, the fermentation broth is continually monitored to maintain a pH range of 6-7, referably using ammonium hydroxide for neutralization of the anionic xanthan gum. Other suitable water-soluble organic bases such as water soluble amines and ammonium salts of organic bases may be used. Suitable water soluble amines are primary amines, e.g. methyl amine, ethyl amine and tromethamine (TRIS buffer); secondary amines, e.g. dimethyl amine, diethylamine and diethanolamine; tertiary amines, e.g. diethylmethyl amine, diethylmethyl 2,2′-dihydroxy amine, and triethanolamine; and quaternary amines, e.g. tetramethyl ammonium hydroxide, tetraethyl ammonium hydroxide, and choline. Suitable water soluble ammonium salts include ammonium carbonate in addition to previously mentioned ammonium hydroxide.

As the viscosity of the broth increases with the amount of the gum produced, frequent monitoring and a corresponding increase in agitation rate assures that all parts of the broth are properly aerated. The criterion of good mixing, well known to those skilled in the polysaccharide fermentation art, is sufficient to produce low-ash xanthan gum of the invention.

In accordance with the procedure of the present invention, fermentation of <u>Xanthomonas</u> <u>campestris</u> NRRL B-l459 is followed by precipitation with a suitable alcohol, preferably isopropyl alcohol and drying.

An alternate procedure also includes addition of ammonium bicarbonate prior to precipitation or washing with isopropyl alcohol

Fermentation media for preparing low-ash xanthan gum comprise dibasic potassium phosphate ($K_2HPO_4$), ammonium nitrate ($NH_4NO_3$), magnesium sulfate heptahydrate ($MgSO_4 \bullet 7H_2O$), Ho-Le trace inorganic salts (see Table I), Hodag K-60, a defoamer, glucose and deionized water. Approximately neutral pH is maintained during the fermentation with ammonium hydroxide. Media also includes additional soluble organic nitrogen source, e.g. NZA type EKC (Sheffield), Casein peptone ALS, and $CaCl_2 \cdot 2H_2O$.

## TABLE 1

### Trace Element Formulations

### Ho-Le Salt Formulation

| Constituent | Mg/L stock | ppm in media |
|---|---|---|
| H3B03 | 285 | 0.05 $B^{+3}$ |
| $MnCl_2 \cdot 4H_2O$ | 1800 | 0.50 $Mn^{+2}$ |
| $FeSO_4 \cdot 7H_2O$ | 2487.2 | 0.50 $Fe^{+2}$ |
| $CuCl_2$ | 26.9 | 0.01 $Cu^{+2}$ |
| $ZnCl_2$ | 20.8 | 0.02 $Zn^{+2}$ |
| $CoCl_2 \cdot 6H_2O$ | 40.4 | 0.01 $Co^{+2}$ |
| $MgMoO_4$ | 19.2 | 0.01 $Mo^{+2}$ |
| Na-tartrate | 2098.0 | ----------- |

Mix each ingredient separately and dissolve in deionized water. Combine ingredients; solution should be clear and yellow. Filter sterilize and dispense at l ml/L.

Table 2 shows preferred media formulations for production of low-ash xanthan gum.

## TABLE 2

| | |
|---|---|
| $K_2HPO_4$ | 0.005 - 0.02 wt.% |
| $NH_4NO_3$ | 0.01 - 0.2 wt.% |
| $MgSO_4 \cdot 7H_2O$ | 0.005 - 0.02 wt.% |
| Ho-Le salts | 0.05 - 0.2 ml/L |
| Hodag, K-60 | 0.03 - 0.12 wt.% |
| NZA, type EKC | 0 - 0.06 wt.% |
| Casein peptone, ALS | 0 - 0.01 wt.% |
| Glucose | 3 - 6 wt.% |
| $CaCl_2 \cdot 2H_2O$ | 0 - 10 ppm $Ca^{+2}$ |
| Deionized Water | to 100% |

According to the procedure of the present invention, all ingredients other than glucose are weighed and dissolved in deionized water and pH is adjusted to about 7. The solution is introduced to a fermentor and accessory equipment and headplate are installed. The fermenter and contents are sterilized at l20°C for l5 minutes. A solution of glucose is prepared with deionized water and sterilized at l20°C for l5 minutes.

*Xanthomas campestris* growth cultures described above and the glucose solution are added to the sterilized fermenter using a peristaltic pump to prevent contamination. Fermentation was allowed to proceed for ap-

EP 0 511 784 A1

proximately 100 hours at an agitation speed of 400-700 rpm and an air flow rate of 10-20 liters/min.

Following the general procedures described above, media having the following constituent amounts were used to prepare low-ash xanthan gum.

## EXAMPLES 1-4

| Constituent | 1 | 2 | 3 | 4 |
|---|---|---|---|---|
| $K_2HPO_4$ | 0.01% | 0.01% | 0.01% | 0.01% |
| $NH_4NO_3$ | 0.021% | 0.021% | 0.10% | 0.10% |
| $MgSO_4 \cdot 7H_2O$ | 0.01% | 0.01% | 0.01% | 0.01% |
| Ho-Le salts | 0.1 ml/L | 0.1 ml/L | 0.1 ml/L | 0.1 ml/L |
| Hodag, K-60 | 0.06% | 0.06% | 0.06% | 0.06% |
| NZA, type EKC | 0.008% | – | 0.03% | 0.03% |
| Casein peptone, ALS | – | 0.005% | – | – |
| Glucose | 3.0% | 3.0% | 3.0% | 6.0% |
| $CaCl_2 \cdot 2H_2O$ | – | – | 5 ppm $Ca^{+2}$ | 5 ppm $Ca^{+2}$ |
| Deionized Water | 100% | 100% | 100% | 100% |
| pH @ 6.7 w/$NH_4OH$ | 210 ml | 255 ml | 400 ml | 780 ml |
| | | | | |
| Fermentation Time | 95 hr | 141 hr | 96 hr | 136 hr |
| Final pH | 6.7 | 6.7 | 7.6 | 6.8 |
| Final Viscosity (cP) | 3,650 | 1,290 | 1,850 | 2,500 |
| Final Yield | 1.69% | 1.41% | 2.13% | 2.50% |

Following fermentation according to each of Examples 1, 2, 3 and 4, the low ash xanthan gum is recovered by the addition of 2.5 volumes of isopropanol and drying the collected precipitate at 140°F for 4 hours.

Examples 5 and 6 describe typical fermentation procedures well known in the art. The media used to prepare xanthan gum included tap water and KOH for pH control, both of which were different from the media used in Examples 1-4.

4

## EXAMPLES 5-6

| Constituent | 5 | 6 |
|---|---|---|
| $Na_2HPO_4$ | 0.026% | – |
| $NH_4NO_3$ | 0.106% | – |
| NZA, type EKC | 0.031% | – |
| Hodag, K-60 | 0.013% | – |
| $FeSO_4 \bullet 7H_2O$ | 5 mg/1 (1 ppm $Fe^{++}$) | – |
| Corn Syrup | 6.0% | – |
| Tap Water | 100% | – |
| $K_2HPO_4$ | – | 0.5% |
| $MgSO_4 \bullet 7H_2O$ | – | 0.02% |
| Urea | – | 0.09% |
| Dextrose | – | 6.0% |
| Distillers dried solubles* | – | 0.8% |
| SAG 5693 | – | 0.006% |
| Fermentation time | 115 hr | 143 hr |
| Final pH | 6.6 (w/KOH) | ~7 (w/$NH_4OH$) |
| Final viscosity (cP) | 5,100 | 1,030 |
| Final yield | 4.39% | 1.26% |

*Aqueous extract of distiller's dried solubles: 10% solution of distiller's dried solubles (solulac®, which contains 70% distiller's dried solubles). Add 4% diatomaceous earth and filter through Whatman filter paper. Use filtered material at 0.8% usage level.

Composition analysis and fermentation results of low-ash xanthan gum versus two xanthan gum controls show the following:

Fermentation

| Results | 1 | 2 | 3 | 4 | 5 | 6 |
|---|---|---|---|---|---|---|
| % carbon source | 3.0 | 3.0 | 3.0 | 6.0 | 6.0 | 6.0 |
| % Yield | 1.69 | 1.41 | 2.13 | 2.50 | 4.39 | 1.26 |
| % C.E. | 56 | 47 | 71 | 42 | 73 | 21 |

Product

| composition | 1 | 2 | 3 | 4 | 5 | 6 |
|---|---|---|---|---|---|---|
| % solids | 91.80 | 91.42 | 93.19 | 93.76 | 86.05 | 94.43 |
| % ash | 0.66 | 0.96 | 0.94 | 0.53 | 9.88 | 14.93 |

The results show that neutralization with $NH_4OH$ and low levels of inorganic ions are required to maintain ash levels below l%.

Solids are measured by drying a sample to constant weight over 3 hours at l05°C. Ash content is determined by ashing a sample at 600°C for 8 hours.

Product

| Properties | 1 | 2 | 3 | 4 | 5 | 6 |
|---|---|---|---|---|---|---|
| Viscosity (cP) | 1280 | 362 | 2550 | 1280 | 1650 | 232 |

Viscosity of a 0.25% w/v solution in standard tap water measured using a Brookfield LVT viscometer 3 rpm, spindle #2. Standard tap water is a solution of l000 ppm sodium chloride and l47 ppm calcium chloride dihydrate dissolved in l liter of deionized water.

Low-ash xanthan gum of the present invention has high viscosity and low ash residue upon combustion.

EXAMPLE 7

The level of ash can be lowered by adding ammonium salts of organic acids to the final fermentation broth prior to recovery by precipitation. Alternatively these salts may be added to the alcohol used for precipitation or washing of the xanthan gum fibers. To the final fermentation broth of Example #4, 32 g of ammonium bicarbonate ($NH_4HCO_3$) were added per kilogram of broth. The treated broth was pasteurized and precipitated with 2.5 volumes of isopropyl alcohol. The xanthan fibers were collected, dried and milled. The viscosity of a 0.25% gum solution in tap water, solids and ash contents of the recovered sample were measured.

| Viscosity (cP) | % Solids | % Ash |
|---|---|---|
| 1720 | 95.12 | 0.36 |

The addition of $NH_4HCO_3$ effectively reduced the ash content of the recovered product from 0.56 (Example #4) to 0.36%.

Greater reductions in ash content are observed when adding $NH_4HCO_3$ to broths containing higher initial

levels of inorganic ions. For example, the products of Example 3 have 0.87% ash, whereas Example 3 with $NH_4HCO_3$ treatment has 0.5l% ash, a 4l% decrease in ash content.

Low ash xanthan gum is particularly suited for use as a binder in preparing ceramic compositions

Ceramic materials are shaped or formed by three major processes; isostatic pressing, extrusion, and injection molding. For these and other formation processes, it is important that the ceramic composition is semi-plastic and remains homogeneous. The compositions comprise friable clusters of granular material such as clays, alumina and other oxide minerals, and binders. Binders are added to ceramic compostions to enhance the semi-plastic property and homegeneity. In addition to strengthening and giving the composition the proper flow properties, the binder must burn out upon firing leaving the minimum amount of residue or ash.

An exemplary plastic refractory ceramic composition using low-ash xanthan gum is prepared, for example, by forming a mixture of tabular alumina, calcined alumina, kyanite, chromic oxide, phosphoric acid, monoaluminum phosphate, bentonite, boric acid, and low ash xanthan gum. Sufficient water is added to obtain a granular refracting mix suitable for formation of ceramic material.

Low-ash xanthan gum is a superior substitute for methylcellulose, a binder commonly used in the ceramic industry. (Davis et al., U.S. Patent 4,952,534). Low-ash xanthan gum provides higher viscosity than methylcellulose and less is required to prepare a suitable plastic refractory ceramic composition.

**Claims**

1. A process for preparing low-ash xanthan gum which comprises:
   (a) growing <u>Xanthomonas campestris</u> in a deionized water medium comprising a fermentable carbohydrate, a nitrogen source, and appropriate other nutrients;
   (b) controlling fermentation broth pH with a water soluble organic base; and
   (c) precipitating the gum with alcohol.

2. The process of Claim 1 wherein the pH is between about 6 and about 7.

3. The process of Claim 1 wherein the alcohol is isopropyl alcohol.

4. The process of Claim 1 wherein the gum contains ash at a level of less than about 2%.

5. The process of Claim 1 wherein an organic acid ammonium salt is added to the broth prior to precipitation of the gum with alcohol.

6. The process of Claim 1 wherein an organic acid ammonium salt is added to the gum following precipitation.

7. The process of Claim 1 wherein the water soluble organic base is ammonium hydroxide.

8. A product prepared according to the process of Claim 1.

9. A plastic refractory ceramic composition comprising friable clusters of granular material and the low-ash xanthan gum of Claim 8.

European Patent
Office

**EUROPEAN SEARCH REPORT**

Application Number

| DOCUMENTS CONSIDERED TO BE RELEVANT | EP 92303687.5 |
|---|---|

| Category | Citation of document with indication, where appropriate, of relevant passages | Relevant to claim | CLASSIFICATION OF THE APPLICATION (Int. Cl.5) |
|---|---|---|---|
| D,X | US - A - 4 375 512 (RICHMON) * Claims * | 1,8 | C 12 P 19/06 C 04 B 35/66 |
| X | US - A - 4 263 399 (EMPEY et al.) * Claims * | 1,8 | |
| X | US - A - 4 328 310 (WEISROCK) * Abstract * | 1,8 | |
| D,X | US - A - 4 952 534 (DAVIS et al.) * Claims * | 9 | |
| | | | **TECHNICAL FIELDS SEARCHED (Int. Cl.5)** |
| | | | C 12 P C 04 B |

The present search report has been drawn up for all claims

| Place of search | Date of completion of the search | Examiner |
|---|---|---|
| VIENNA | 03-08-1992 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04011)